# EUROPEAN PATENT APPLICATION

(11) **EP 3 725 305 A1**
(43) Date of publication of application: **21.10.2020**
(21) Application number: 19169785.3
(22) Date of filing: 17.04.2019
(51) Int. Cl.: A61K 9/20, A61K 9/28, A61K 31/519

(54) **PHARMACEUTICAL COMPOSITION CONTAINING BARICITINIB HYDROBROMIDE**

(71) Applicant: Zentiva K.S., 102 37 Praha 10 (CZ)
(72) Inventor: Kluk, Anna, 10-691 Olsztyn (PL); Tieger, Eszter, Magyarlak (HU); Podgórna, Karolina, 13000 Praha 3 (CZ); Schongut, Marek, 14000 Praha 4 (CZ)
(74) Representative: Hartvichova, Katerina

(57) **Abstract**

The invention relates to a composition for a pharmaceutical solid drug form, which comprises baricitinib hydrobromide in the amount of 2.5 % w/w or more, relative to the total weight of the composition, a filler, and at least one additional pharmaceutically acceptable excipient. Such composition has a number of technological advantages.

The additional pharmaceutically acceptable excipients may include disintegrants and lubricants.

## Description

### Field of Art

The present invention relates to a composition of a pharmaceutical solid dosage form, containing 2-(3-(4-(7H-pyrrolo[2,3-d]pyrimidin-4-yl)-1H-pyrazol-1-yl)-1-(ethylsulfonyl)-azetidin-3-yl)acetonitrile, known as baricitinib (formula I), 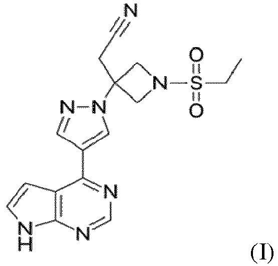 in the form of hydrobromide salt. The invention further relates to a manufacturing process of the pharmaceutical solid dosage forms, containing baricitinib hydrobromide.

### Background Art

Baricitinib is a therapeutic drug substance belonging to the group of Janus kinase inhibitors (JAK inhibitors) and it is used for treating rheumatoid arthritis (a disease causing inflammation of the joints). Baricitinib is available in the form of film coated tablets, available under the commercial name of Olumiant 4 mg and 2 mg. Olumiant is used in patients with moderate to severe arthritis, when standard treatment with disease-modifying anti-rheumatic drugs (also known as 'DMARDs') has not worked well enough or if patients cannot tolerate them.

Baricitinib has been fully described for the first time in WO2009114512 (Incyte Corp.), where both baricitinib alone and its phosphate salts have beed described. However, it has been also mentioned before in WO2007117494, as an analogue of ruxolitinib, developed also by the same company. EP2288610A described specifically baricitinib as such as well as its trifluoroacetic and phosphate salts. Synthesis of baricitinib is described in WO2009114512. Preparation of amorphous form of baricitinib has been disclosed in WO2015145286 from Sun Pharmaceutical Industries. The same company disclosed also the crystalline forms of API in WO2015166434, covering the crystalline form of baricitinib free base Form I, present in the marketed product Olumiant®. Further polymorphic form of baricitinib were described in WO2016141891, including free base Form I, acetic acid solvate and three forms of baricitinib phosphate. Yet further salts of baricitinib have been described in Zentiva's patent applications: crystalline baricitinib salts with hydrobromic, hydrochloric, sulfuric, methanesulfonic, ethanesulfonic, p-toluenesulfonic, L-tartaric and fumaric acids were disclosed in CZ PV 2016-705 and EP3321267, while baricitinib phosphate forms - monophosphate A-G and hemiphosphate forms I and II - were disclosed in CZ PV 2016-816.

The commercial product Olumiant® 2 mg and 4 mg contains crystalline form I of baricitinib base. The manufacturing process of this original drug product consists of blending, dry granulation, blending, compression and film-coating. Final drug form is a film coated tablet. Olumiant 2 mg film-coated tablets have a form of light pink, 9.0 x 7.5 mm oblong tablets, while the higher 4 mg strength is available in the form of medium pink, 8.5 mm round tablets of the weight of about 200 mg. Sizes of both strengths result in Olumiant not having a proportional composition, and since the concentration of API in the higher strength tablet is very low (about 2 % w/w) and in 2 mg tablets even about two times lower, the original drug form needs to be assessed as non-standard, requiring additional operations during manufacturing process in order to provide sufficient content uniformity in the tablets and later - full production-scale data for validation process, according to the *Guideline on Process Validation for Finished Products (EMA*/*CHMP*/*CVMP*/*QWP*/*BWP*/*70278*/*2012-Rev1,Corr.1).*

There is a strong need in the field to provide a suitable pharmaceutical composition which could be formulated as proportional tablets being considered as a standard drug product without requiring additional care during manufacturing to provide sufficient content uniformity.

### Disclosure of the invention

The present invention relates to a composition for a pharmaceutical solid dosage form and manufacturing process of the said solid dosage form containing hydrobromide (HBr) salt of baricitinib in crystalline form.

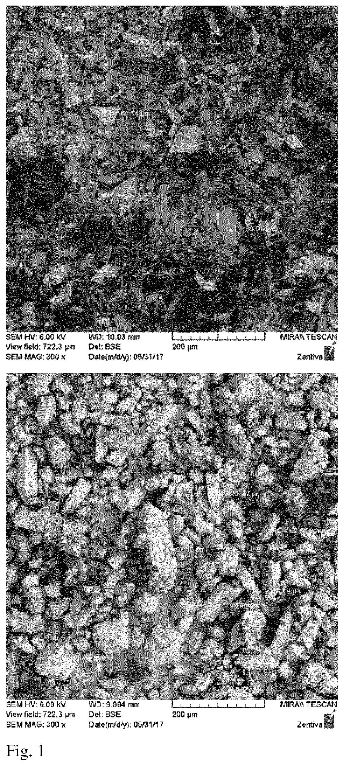

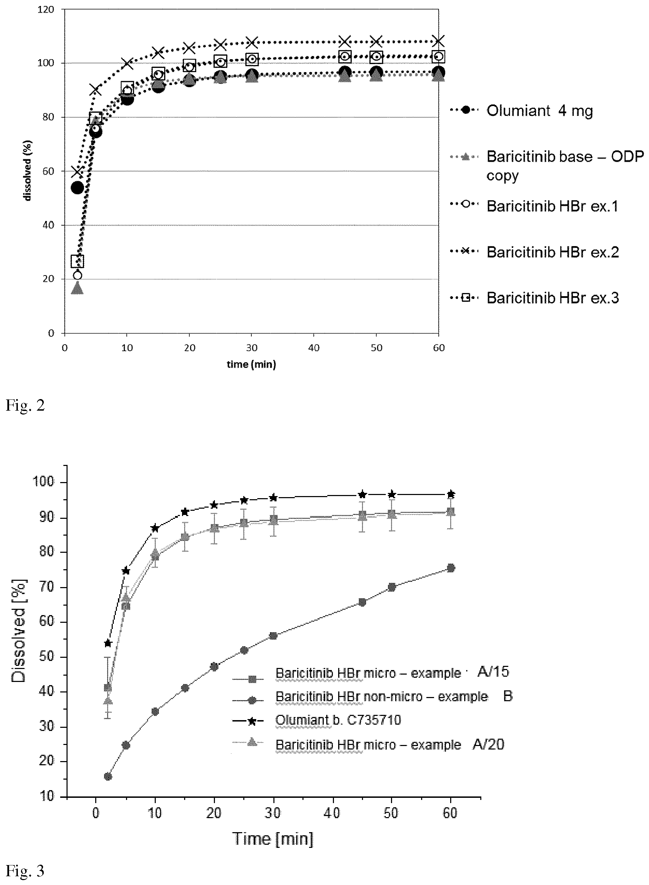

Within the framework of the invention, it has been surprisingly found that using a composition comprising baricitinib hydrobromide may significantly improve the manufacturing process of the final drug form (solid dosage form). The inventors have observed that baricitinib hydrobromide has a solid crystalline form which is suitable for easy grinding of the drug substance to the micronized form. Figure 1 shows the SEM photos of baricitinib hydrobromide and baricitinib base (the form used in the original drug product), and the comparison confirms that unlike baricitinib base, baricitinib hydrobromide crystals are regular and do not form thin sticky plates. The crystalline structure of baricitinib hydrobromide facilitates its regular distribution within the pharmaceutically acceptable excipients, and thus improves the content uniformity in the final tablets. Moreover, it has been surprisingly observed that micronization of the API reduces stickiness of the final composition, which is helpful during both tableting process and dissolution test - a faster dissolution profile is registered for tablets with micronized API in comparison with tables with non-micronized API (Fig.3.).

Another advantage is that baricitinib hydrobromide has a higher molecular weight than baricitinib base (452.33 g/mol and 371.42 g/mol, accordingly), which allows to increase the amount of API in the composition from 2.00 % w/w to 2.44 % w/w, which improves content uniformity in the final drug form. However, 2.44 % w/w is still a too low concentration to consider the final drug form as standard one (the limit concentration is ≥2.5 % w/w). It was surprisingly found that for baricitinib hydrobromide, reduction of the tablet mass by about 4.8 mg (from the core weight 200 mg to the final core weight 195.2 mg) allows to achieve a final API content ≥ 2.5 % w/w, and thus the final drug form is considered as a standard one, while for baricitinib base the same requirement would lead to reduction of the core weight to 160 mg. A tablet having 160 mg tablet core with baricitinib hydrobromide has 3.04 % w/w of the active ingredient in the core, which is a much safer concentration for the manufacturing process from the homogeneity point of view. Combination of all these factors allows to manufacture the solid dosage form (final drug form) in a standard manufacturing process and produce both tablet strengths in a proportional way, while still ensuring adequate homogeneous distribution of baricitinib hydrobromide in the formulation (complying with content uniformity requirements) and an immediate release profile.

The present invention relates to a composition for a pharmaceutical solid drug form, which comprises baricitinib hydrobromide in the amount of 2.5 % w/w or more, relative to the total weight of the composition, and a filler and at least one additional pharmaceutically acceptable excipient. In some embodiments, the additional pharmaceutically acceptable excipient is a disintegrant.

In a preferred embodiment, the amount of baricitinib hydrobromide is 2.50 - 3.50 % by weight of the total weight of the composition, which enables to provide a proper product quality within standard manufacturing process, not requiring full production-scale data for validation process.

In another preferred embodiment the claimed composition comprises baricitinib hydrobromide, with particle size distribution wherein 90 % of particles or more is smaller than 10 µm (d(0.9) < 10 µm). The % is a cumulative undersize distribution, i.e. 90% means that 90 particles from 100 particles measured are smaller than 10 µm. Such particle size distribution can be obtained by techniques well known in the art. Particularly preferred is micronization process which further facilitates the homogenous distribution of baricitinib hydrobromide within the formulation and protects the composition from sticking, thus facilitating its further processing.

A preferred composition comprises:
- 2.5-3.5 % w/w of (preferably micronized) baricitinib hydrobromide (preferably particle size distribution of d(0.9) < 10 um),
- 85.5-94.5 % w/w of one or more filler(s),
- the balance being completed by at least one pharmaceutically acceptable excipients selected from disintegrants, lubricants, binders, colourants, flavours, glidants, preservatives.

A particularly preferred composition comprises:
- 2.5-3.5 % w/w of (preferably micronized) baricitinib hydrobromide (preferably particle size distribution of d(0.9) < 10 um),
- 2-10 % w/w of one or more disintegrants,
- 85.5-94.5 % w/w of one or more filler(s),
- 1-3% w/w of lubricant,
- the balance being further completed by pharmaceutically acceptable excipients such as binders, colourants, flavours, glidants, preservatives, optionally in the amount of up to 2%.

Preferably, the filler is selected from lactose, mannitol, starch and its derivatives, powdered cellulose, microcrystalline cellulose and mixtures thereof.

Preferably, the disintegrant is selected from croscarmellose sodium and sodium starch glycolate.

Preferably, the lubricant is magnesium stearate, sodium stearyl fumarate or glyceryl behenate.

Furthermore, the present invention provides a solid dosage form comprising the composition of the invention.

The solid dosage form may be a tablet, uncoated or coated, wherein the core of the tablet is composed of the composition of the invention, or a capsule. The coated tablets further contain pharmaceutically acceptable cosmetic coating, preferably the cosmetic coating forms 2-6 % by weight of the total weight of the tablet.

The cosmetic coating may comprise:
- at least one polymeric excipient (e.g., hypromellose, hydroxypropyl cellulose, polyvinyl alcohol, methyl cellulose, ethyl cellulose).
- optionally plasticizers (e.g., polyethylene glycol, triacetin, triethyl citrate or mineral oil)
- optionally additives such as fillers and glidants (e.g., talc, kaolin, lecithin),
- optionally surfactants (e.g., sodium lauryl sulfate, polysorbates)
- optionally conventional pharmaceutically acceptable pigments (e.g., iron oxides, aluminum-based compounds, titanium dioxide) or natural or synthetic dyes.

In some embodiments, the solid dosage form are immediate release capsules or immediate release tablets, either coated or uncoated, as coating does not have any significant influence on dissolution profile.

The present invention also relates to the manufacturing process of pharmaceutical composition, containing baricitinib hydrobromide. The proposed manufacturing process may comprise baricitinib hydrobromide milling, sieving or micronization; then the step of blending with excipients, followed by compacting, dry or wet granulation; and then either tabletting and coating of the core or filling powder or granules into capsules.

The invention is illustrated by the following examples. The examples, which illustrate the preparation of final dosage drug forms, containing baricitinib hydrobromide, should not be construed as limiting the scope of the invention.

### Brief description of Drawings

Fig. 1: Crystalline structure of baricitinib base (left) and baricitinib HBr salt (right)
Fig. 2: Dissolution profiles of the Olumiant, original drug product copy with baricitinib base and exemplary prototypes with baricitinib HBr salt.
Fig. 3: Dissolution profiles of baricitinib HBr tablets with micronized and non-micronized API.

### Examples

### Methods

SEM measurement - The electron microscope images were obtained using a MIRA II device, Tescan, Czech Republic, 2006. As the cathode, W fiber with the applied voltage of 7 kV was used with a YAG scintillation detector positioned in the optical axis under the pole piece of the objective (BSE). The measurement was carried out under high vacuum. The sample was put onto a carbon tape and coated with Pt in a vacuum sputter before the measurement.

Measurement of particle size - The particle size was determined directly from SEM photos using NIS elements, from at least 100 particles measured. Particle size distribution reported based on Feret's diameter.

Dissolution test measurement - Paddle apparatus, peak vessels, medium: 500 mL 72mM phosphate buffer, pH 6.8, paddle speed: 50 rpm + 75 rpm from the time point of 45 min. Content uniformity measurement - according Ph.Eur. 2.9.40, Ph.Eur 9th edition.
% are wt. %, unless otherwise specified.

### Example 1

| **Composition** | **Tablet [mg]** | **Tablet core [%]** | **FCT [%]** | **Quality** |
|---|---|---|---|---|
| **Baricitinib HBr,** micronized d(0.9)< 10 µm | 4.871 | 3.04 % | 2.93% | In-house specification |
| **Mannitol** | 24.000 | 15.00% | 14.42% | Ph.Eur. |
| **Cellulose microcrystalline** | 121.529 | 75.96% | 73.03% | Ph.Eur. |
| **Croscarmellose sodium** | 8.000 | 5.00 % | 4.81% | Ph.Eur. |
| **Magnesium stearate** | 1.600 | 1.00% | 0.96% | Ph.Eur. |
| **Total cores:** | **160.000** | **100%** | | |
| **Cosmetic coating (HPMC, HPC, PEG 6000, talc, titanium dioxide, iron oxide red)** | **6.400** | - | 3.85% | Ph.Eur. |
| **Total FCT** | **166.400** | | **100.00%** | |

### Preparation:

Micronized baricitinib hydrobromide (baricitinib HBr) was weighted and then mixed with the whole amount of mannitol for 10 min. Then cellulose microcrystalline and crosscarmellose sodium were added and the mixture was blended for another 5 min. Ready mixture ready for compression (MRC) was subjected to dry compaction with a force of 20 kN and sieved through 1.25 mm, then mixed with magnesium stearate for 3 min. MRC was compressed to the final core weight of 160 mg with precompression force of 2kN and main compression force of 15kN. Cores were subsequently coated with cosmetic coating up to the weight gain 4 %. The final hardness of film coated tablets (FCT) was 96 N and disintegration time 3 min. Dissolution profile is shown in Figure 2.

Optionally, magnesium stearate may be replaced by any other lubricant, preferably by glyceryl behenate or sodium stearofumarate, both in concentration 1-3% (compensated with lower amount of microcrystalline cellulose).

### Example 2

| **Composition** | **Tablet [mg]** | **Tablet core [%]** | **FCT [%]** | **Quality** |
|---|---|---|---|---|
| **Baricitinib HBr,** micronized d(0.9)< 10 µm | 4.871 | 3.04 % | 2.93% | In-house specification |
| **Cellulose microcrystalline** | 145.529 | 90.96% | 87.46% | Ph.Eur. |
| **Croscarmellose sodium** | 8.000 | 5.00 % | 4.81% | Ph.Eur. |
| **Magnesium stearate** | 1.600 | 1.00% | 0.96% | Ph.Eur. |
| **Total cores:** | **160.000** | **100%** | | |
| **Cosmetic coating (HPMC, HPC, PEG 6000, talc, titanium dioxide, iron oxide red)** | **6.400** | - | 3.85% | Ph.Eur. |
| **Total FCT** | **166.400** | | **100.00%** | |

### Preparation:

Micronized baricitinib HBr was weighted and then mixed with the whole amount of microcrystalline cellulose for 10 min. Then crosscarmellose sodium was added and the mixture was blended for another 5 min. Powder mixture was then lubricated with a half of amount of magnesium stearate for 3 min. Ready MRC was subjected to dry compaction with a force of 20 kN and sieving through 1.25 mm sieve, then mixed with the rest of magnesium stearate (0.5%) for another 3 min. MRC was compressed to the final core weight of 160 mg with precompression force of 2kN and main compression force of 15kN. Cores were subsequently coated with cosmetic coating up to the weight gain 4%. The final hardness of FCT was 80 N and disintegration time 2.5 min. Dissolution profile is shown in Figure 2.

Optionally, magnesium stearate may be replaced by any other lubricant, preferably by glyceryl behenate or sodium stearofumarate, both in concentration 1-3% (compensated with lower amount of microcrystalline cellulose).

### Example 3

| **Composition** | **Tablet [mg]** | **Tablet core [%]** | **FCT [%]** | **Quality** |
|---|---|---|---|---|
| **Baricitinib HBr,** micronized d(0.9)< 10 µm | 4.871 | 3.04 % | 2.93% | In-house specification |
| **Lactose monohydrate** | 24.000 | 15.00% | 14.42% | Ph.Eur. |
| **Cellulose microcrystalline** | 121.529 | 75.96% | 73.03% | Ph.Eur. |
| **Sodium starch glycolate** | 8.000 | 5.00 % | 4.81% | Ph.Eur. |
| **Magnesium stearate** | 1.600 | 1.00% | 0.96% | Ph.Eur. |
| **Total cores:** | **160.000** | **100%** | | |
| **Cosmetic coating (HPMC, HPC, PEG 6000, talc, titanium dioxide, iron oxide red)** | **6.400** | - | 3.85% | Ph.Eur. |
| **Total FCT** | **166.400** | | **100.00%** | |

### Preparation:

Micronized baricitinib HBr was weighted and then mixed with the whole amount of Lactose monohydrate for 10 min. Then cellulose microcrystalline and sodium starch glycolate were added and the mixture was blended for another 5 min, and then with a half amount of magnesium stearate for additional 3 min. Ready MRC was subjected to dry compaction with a force of 20kN and sieving through 1.25 mm sieve, then mixed with a half of amount of magnesium stearate for 3 min. MRC was compressed to the final core weight of 160 mg with precompression force of 2kN and main compression force of 15kN. Cores were subsequently coated with cosmetic coating up to the weight gain 4%. The final hardness of FCT was 90 N and disintegration time 2 min 40s. Dissolution profile is shown at Figure 2.

Optionally, magnesium stearate may be replaced by any other lubricant, preferably by glyceryl behenate or sodium stearofumarate, both in concentration 1-3% (compensated with lower amount of microcrystalline cellulose).

The compositions of Example 1, Example 2 and Example 3 were compared with a baricitinib base composition (ODP copy 1:1). The results are shown in Table 1 and Figure 2.

**Table 1 Assay and content uniformity of Olumiant copy and prototypes with baricitinib HBr.**

| **Core composition [wt.%]** | **ODP copy 1:1** | **example 1** | **example 2** | **example 3** |
|---|---|---|---|---|
| Baricitinib base | 2.00% | - | - | - |
| Baricitinib HBr. micronized d(0.9)< 10 um | - | 3.04 % | 3.04 % | 3.04 % |
| Mannitol | 15.00% | 15.00% | - | - |
| Lactose monohydrate | - | - | - | 15.00% |
| Cellulose microcrystalline | 77.00% | 75.96% | 90.96% | 75.96% |
| Crosscarmellose sodium | 5.00% | 5.00% | 5.00 % | - |
| Sodium starch glycolate | - | - | | 5.00 % |
| Magnesium stearate | 1.00% | 1.00% | 1.00% | 1.00% |
| **Theoretical core weight** | **200.00 mg** | **160.00 mg** | **160.00 mg** | **160.00 mg** |
| **Real core weight - average** | 202.45 | 163.75 | 161.4 | 162.7 |
| **Real core weight - SD** | 3.75 | 1.83 | 3.14 | 2.27 |
| **Real core weight - RSD** | 1.9% | 1.1% | 1.9% | 1.4% |
| **Assay (declared API dose specification: 95-105%)** | **97.19** | **99.54** | 99.84 | **100.4** |
| **Content Uniformity (AV)** | **6.6** | **4.3** | **5.1** | **5.9** |

### Example 4

| **Core Composition [wt.%]** | **ODP copy 1:1** | **example A** | **example B** |
|---|---|---|---|
| **Baricitinib base,** | 2.00% | | |
| **Baricitinib HBr,** micronized d(0.9)< 10 µm | | 3.04% | |
| **Baricitinib HBr,** non-micronized d(0.9)< 200 µm | | | 3.04% |
| **Mannitol** | 15.00% | 15.00% | 15.00% |
| **Cellulose microcrystalline** | 77% | 75.96% | 75.96% |
| **Crosscarmellose sodium** | 5.00% | 5.00% | 5.00% |
| **Magnesium stearate** | 1.00% | 1.00% | 1.00% |

### Preparation:

Micronized or non-micronized baricitinib HBr was weighted and then mixed with the whole amount of Mannitol for 10 min. Then cellulose microcrystalline and Crosscarmellose sodium were added and the mixture was blended for another 5 min, and then with a half amount of magnesium stearate for additional 3 min. Ready mixture was subjected to dry compaction with a force of 20kN and sieving through 1.25 mm sieve, then mixed with a half of amount of magnesium stearate for 3 min. MRC was compressed with precompression force of 2 kN and main compression force of 15 kN (in case of Example A, two compression forces were used - 15 kN and 20 kN). Dissolution profiles are shown in Figure 3.

## Claims

1. A composition for a pharmaceutical solid drug form, **characterized in that** it comprises baricitinib hydrobromide in the amount of 2.5 % w/w or more, relative to the total weight of the composition, a filler, and at least one additional pharmaceutically acceptable excipient.

2. The composition according to claim 1, wherein at least 90% of particles of baricitinib hydrobromide salt is smaller than 10 µm.

3. The composition according to claim 2, wherein baricitinib hydrobromide is micronized.

4. The composition according to any one of the preceding claims, wherein the composition contains from 2.5 to 3.5 % w/w of crystalline baricitinib hydrobromide salt.

5. The composition according to any one of the preceding claims, wherein the filler is selected from lactose, mannitol, starch and its derivatives, powdered cellulose, microcrystalline cellulose and mixtures thereof.

6. The composition according to any one of the preceding claims, wherein the at least one additional pharmaceutically acceptable excipient is a disintegrant.

7. The composition according to claim 6, wherein the disintegrant is selected from croscarmellose sodium and sodium starch glycolate.

8. The composition according to any one of the preceding claims, which further comprises a lubricant, preferably selected from magnesium stearate, sodium stearyl fumarate and glyceryl behenate.

9. The composition according to any one of the preceding claims, which comprises
- 2.5-3.5 % w/w of baricitinib hydrobromide,
- 85.5-94.5 % w/w of one or more filler(s),
- the balance being further completed by pharmaceutically acceptable excipients selected from disintegrants, lubricants, binders, colourants, flavours, glidants, preservatives.

10. The composition according to any one of the preceding claims, which comprises:
- 2.5-3.5 % w/w of baricitinib hydrobromide,
- 2-10 % w/w of one or more disintegrants,
- 85.5-94.5 % w/w of one or more filler(s),
- 1-3% of lubricant,
- the balance being further completed by pharmaceutically acceptable excipients such as binders, colourants, flavours, glidants, preservatives.

11. A solid dosage form, comprising the composition according to any one of the preceding claims, which is selected from uncoated tablets, coated tablets, capsules.

12. The solid dosage form according to claim 11, which is a coated tablet and has the following composition of the core:
- 2.5-3.5 % w/w of baricitinib hydrobromide,
- 85.5-94.5 % w/w of one or more filler(s),
- the balance being further completed by pharmaceutically acceptable excipients selected from disintegrants, lubricants, binders, colourants, flavours, glidants, preservatives,
and further comprising from 2 to 6 % of a pharmaceutically acceptable coating by weight, relative to the total weight of the tablet.

13. The solid dosage form according to claim 11, which is a coated tablet and has the following composition of the core:
- 2.5-3.5 % w/w of baricitinib hydrobromide,
- 2-10 % w/w of one or more disintegrants,
- 85.5-94.5 % w/w of one or more filler(s),
- 1-3% w/w of lubricant,
- the balance being further completed by pharmaceutically acceptable excipients such as binders, colourants, flavours, glidants, preservatives,
and further comprising from 2 to 6 % of a pharmaceutically acceptable coating by weight, relative to the total weight of the tablet.

14. The solid dosage form according to any one of claims 11 to 13, wherein the pharmaceutically acceptable coating comprises at least one polymeric excipient, preferably selected from hypromellose, hydroxypropyl cellulose, polyvinyl alcohol, methyl cellulose, ethyl cellulose, and optionally one or more of further excipients selected from plasticizers, fillers and glidants, pharmaceutically acceptable pigments, and natural or synthetic dyes.

15. A process for preparation of a composition according to any one of claims 1 to 10, comprising the steps of:
- milling, sieving, or micronization of baricitinib hydrobromide,
- blending with excipients,
- compaction or dry or wet granulation,
- tabletting and optionally coating of the core, or filling the powder or granules into capsules.
